(19) Europäisches Patentamt
European Patent Office
Office européen des brevets

(11) Veröffentlichungsnummer: **0 233 150 B1**

# (12) EUROPÄISCHE PATENTSCHRIFT

(45) Veröffentlichungstag der Patentschrift: **11.11.92**

(51) Int. Cl.$^5$: **C07D 401/04**

(21) Anmeldenummer: **87810068.4**

(22) Anmeldetag: **04.02.87**

## (54) Verfahren zur Herstellung von 2-(Imidazolin-2-yl)-pyridin- und -chinolin-3-carbonsäuren.

(30) Priorität: **10.02.86 CH 515/86**

(43) Veröffentlichungstag der Anmeldung:
**19.08.87 Patentblatt 87/34**

(45) Bekanntmachung des Hinweises auf die Patenterteilung:
**11.11.92 Patentblatt 92/46**

(84) Benannte Vertragsstaaten:
**AT BE CH DE ES FR GB GR IT LI NL SE**

(56) Entgegenhaltungen:
**EP-A- 0 095 105**

(73) Patentinhaber: **CIBA-GEIGY AG**
**Klybeckstrasse 141**
**CH-4002 Basel(CH)**

(72) Erfinder: **Szczepanski, Henry, Dr.**
**Bodenmatt**
**CH-4323 Wallbach(CH)**
Erfinder: **Dürr, Dieter, Dr.**
**Brändelistalweg 16**
**CH-4103 Bottmingen(CH)**

EP 0 233 150 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 2-(Imidazolin-2-yl)-pyridin- und -chinolin-3-carbonsäuren der Formel I

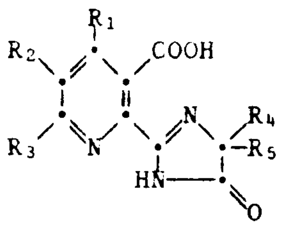

(I)

in welcher $R_1$ Wasserstoff oder $C_1$-$C_4$-Alkyl, $R_2$ Wasserstoff, Halogen, $C_1$-$C_4$-Alkyl, Hydroxy-$C_1$-$C_4$-alkyl, $C_1$-$C_4$-Alkoxy, Phenyl, oder Phenyl-$C_1$-$C_4$-alkyl oder einfach durch $C_1$-$C_4$-Alkyl-$C_1$-$C_4$-alkoxy oder Halogen substituiertes Phenyl oder Phenyl-$C_1$-$C_4$-alkyl, $R_3$ Wasserstoff, $C_1$-$C_4$-Alkyl, Phenyl, Phenyl-$C_1$-$C_4$-alkyl oder durch $C_1$-$C_4$-Alkyl, $C_1$-$C_4$-Alkoxy oder Halogen einfach substituiertes Phenyl oder Phenyl-$C_1$-$C_4$-alkyl, $R_2$ und $R_3$ zusammen 1,3-Butadienylen, das durch Halogen, $C_1$-$C_4$-Alkyl, $C_1$-$C_4$-Alkoxy, $C_1$-$C_4$-Halogenalkyl, $C_1$-$C_4$-Alkylsulfonyl, Nitro, Cyano, Phenyl, Phenoxy oder durch $C_1$-$C_4$-Alkyl, $C_1$-$C_4$-Alkoxy oder Halogen einfach substituiertes Phenyl oder Phenoxy substituiert sein kann und $R_4$ und $R_5$ unabhängig voneinander $C_1$-$C_4$-Alkyl bedeuten.

Die Verbindungen der Formel I besitzen herbizide Wirkung und können zur Bekämpfung von unerwünschtem Pflanzenwuchs verwendet werden. Verbindungen dieser Art sind beispielsweise in der publizierten Europäischen Patentanmeldung 0 041 623 und in der US-Patentschrift 4 518 780 beschrieben.

Aus der publizierten Europäischen Patentanmeldung 0 041 623 ist es bekannt, die 2-(Imidazolin-2-yl)-pyridin- und -chinolin-3-carbonsäuren ausgehend von Derivaten der entsprechenden Pyridin- und Chinolin-2,3-dicarbonsäuren und 2-Aminoalkancarbonsäurederivaten herzustellen. Dabei werden als Derivate von Pyridin- und Chinolin-2,3-dicarbonsäuren die Monoester und deren Säurehalogenide, die Monoamide und insbesondere die Anhydride und als 2-Aminoalkancarbonsäurederivate die freien Carbonsäuren, die Amide und insbesondere die Nitrile genannt. So kann beispielsweise die 2-(5-Isopropyl-5-methyl-4-oxoimidazolin-2-yl)-pyridin-3-carbonsäure ausgehend von Pyridin-2,3-dicarbonsäureanhydrid und 2-Amino-2,3-dimethylbutyronitril nach den in Beispielen 1, 2 und 6 der vorgenannten Europäischen Patentanmeldung beschriebenen Verfahrensschritten bezogen auf das eingesetzte Anhydrid in einer Ausbeute von etwa 32 % der Theorie hergestellt werden. In der US-Patentschrift 4 518 780 wird die Herstellung der gleichen Verbindung ausgehend von Pyridin-2,3-dicarbonsäureanhydrid und 2-Amino-2,3-dimethylbutyramid mit einer Ausbeute von 63 % der Theorie bezogen auf eingesetztes Anhydrid beschrieben. Dieses Verfahren wird in der Weise durchgeführt, dass man das Anhydrid und das Aminobutyramid zunächst in Acetonitril zu einem Gemisch der Isomeren Pyridin-2,3-dicarbonsäuremonoamide umsetzt, das Lösungsmittel abtrennt, den Rückstand bei 80 °C mit wässriger Natronlauge behandelt und das gebildete Produkt durch Zugabe von Säure abscheidet. Nach der gleichen Methode wird die 2-(5-Isopropyl-5-methyl-4-oxoimidazolin-2-yl)-6-isopropyl pyridin-3-carbonsäure in einer Ausbeute von 51 % der Theorie und die 2-(5-Isopropyl-5-methyl-4-oxoimidazolin-2-yl)-chinolin-3-carbonsäure in einer Ausbeute von 50 % der Theorie erhalten, wobei die angegebenen Ausbeuten jeweils auf eingesetztes Anhydrid bezogen sind.

Die in den bekannten Verfahren als Ausgangsmaterial verwendeten Anhydride sind hochreaktive Verbindungen, die wegen ihrer Empfindlichkeit gegen Feuchtigkeit nur unter Einhaltung gewisser Vorsichtsmassregeln transportiert und gelagert werden können. Das Verfahren selbst umfasst zwei separat durchzuführende Reaktionsstufen und ist daher relativ aufwendig. Ferner ist das Verfahren auch in bezug auf die damit erreichbaren Ausbeuten unbefriedigend.

Es ist daher das Ziel der vorliegenden Erfindung, ausgehend von gut zugänglichen und leicht handhabbaren Ausgangsmaterialien ein Verfahren zur Herstellung der 2-(2-Imidazolin-2-yl)-pyridin- und -chinolin-3-carbonsäuren der Formel I bereitzustellen, das die Herstellung dieser Verbindungen auf einfache Weise und in guter Ausbeute ermöglicht.

Gemäss vorliegender Erfindung wird vorgeschlagen, die 2-(Imidazolin-2-yl)-pyridin- und -chinolin-3-carbonsäuren der Formel I in der Weise herzustellen, dass man einen Pyridin- oder Chinolin-2,3-dicarbonsäureesterder Formel II

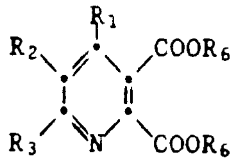

(II)

in welcher $R_1$, $R_2$ und $R_3$ die unter Formel I angegebene Bedeutung haben und $R_6$ $C_1$-$C_8$-Alkyl, Phenyl oder Phenyl-$C_1$-$C_4$-alkyl bedeuten, in einem inerten Lösungsmittel in Gegenwart einer starken Base bei einer Temperatur von Raumtemperatur bis Rückflusstemperatur des Reaktionsgemisches mit einem 2-Aminoalkancarbonsäureamid der Formel III

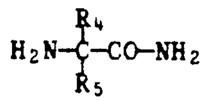

(III)

in welcher $R_4$ und $R_5$ die unter Formel I angegebene Bedeutung haben, umsetzt, das gebildete Salz der 2-(Imidazolin-2-yl)-pyridin- oder -chinolin-3-carbonsäure der Formel I mit Wasser aufnimmt, den pH-Wert der wässrigen Lösung auf 1,5-4,5 stellt und die freie 2-(Imidazolin-2-yl)-pyridin- oder -chinolin-3-carbonsäure der Formel I abtrennt.

Als inerte Lösungsmittel kommen beispielsweise flüssige aromatische Kohlenwasserstoffe und Halogenkohlenwasserstoffe, wie Benzol, Toluol, Chlorbenzol und Xylole, $C_1$-$C_{10}$-Alkanole, insbesondere $C_1$-$C_4$-Alkanole, wie Methanol, Ethanol, Propanol, Isopropanol, n-Butanol, sec.Butanol, tert.Butanol und Isobutanol, etherartige Flüssigkeiten, wie Tetrahydrofuran und Dioxan, sowie stark polare Lösungsmittel, wie Acetonitril, N,N-Dimethylformamid und Dimethylsulfoxid in Betracht. Vorteilhaft werden mit Wasser nicht mischbare Lösungsmittel verwendet, wie Benzol, Toluol, Chlorbenzol oder Xylole.

Als starke Basen, in deren Gegenwart die Umsetzung eines Pyridin-oder Chinolin-2,3-dicarbonsäureesters der Formel II mit einem 2-Aminoalkancarbonsäureamid der Formel III durchgeführt wird, sind Alkalimetallhydroxide und Alkalimetallalkoholate geeignet. Bevorzugte Basen sind Alkalimetallalkoholate, insbesondere solche, die sich von $C_1$-$C_4$-Alkanolen ableiten, wie Natriummethylat, Natriumethylat, Natriumisopropylat und Kalium-tert.butylat. Alkalimetallhydroxide wie Natriumhydroxid und Kaliumhydroxid sind insbesondere bei Verwendung von Alkanolen als inerte Lösungsmittel geeignet. Die Umsetzung kann ferner in Gegenwart von Pottasche in Ethanol durchgeführt werden. Die starke Base wird in der Regel in mindestens equimolarer Menge bezogen auf eingesetzten Pyridin- oder Chinolin-2,3-dicarbonsäureester der Formel II eingesetzt. Bei der Durchführung des erfindungsgemässen Verfahrens hat sich die Verwendung von 1-3 Mol Base pro Mol Pyridin- oder Chinolin-2,3-dicarbonsäureester der Formel II als geeignet erwiesen. Vorzugsweise werden 1,5-2,5 Mol Base pro Mol Pyridin- bzw. Chinolin-2,3-dicarbonsäureester der Formel II verwendet.

Innerhalb des angegebenen Temperaturbereichs von Raumtemperatur bis Rückflusstemperatur des Reaktionsgemisches sind für die Durchführung des erfindungsgemässen Verfahrens Temperaturen von 50-90 °C bevorzugt.

Das Aufnehmen des bei der erfindungsgemässen Umsetzung unmittelbar gebildeten Salzes einer 2-(Imidazolin-2-yl)-pyridin- bzw. -chinolin-3-carbonsäure der Formel I in Wasser kann je nach Art des verwendeten Lösungsmittels und unter Berücksichtigung der Löslichkeit des Salzes in dem jeweils verwendeten Lösungsmittel in verschiedener Weise erfolgen. Normalerweise, insbesondere bei Verwendung von flüssigen aromatischen Kohlenwasserstoffen und Halogenkohlenwasserstoffen, wie Toluol oder Chlorbenzol, scheiden sich die gebildeten Salze praktisch vollständig aus dem Reaktionsgemisch ab. Sie können dann durch Filtration abgetrennt und in Wasser gelöst werden oder aber direkt durch Extraktion mit Wasser in eine wässrige Lösung übergeführt werden. Bei Verwendung von Alkanolen als Lösungsmittel, insbesondere bei Verwendung von niederen Alkanolen, wie Methanol, Ethanol und Isopropanol kann das Reaktionsgemisch direkt mit Wasser verdünnt und weiter verarbeitet werden. Dies gilt auch für die Verwendung von Tetrahydrofuran, Dioxan, Acetonitril, Dimethylformamid und Dimethylsulfoxid. Es ist jedoch auch möglich, die Lösungsmittel ganz oder teilweise durch Destillation zu entfernen und den Rückstand in Wasser aufzunehmen.

Das Freisetzen der 2-(Imidazolin-2-yl)-pyridin- bzw. -chinolin-3-carbonsäuren der Formel I aus der wässrigen Lösung ihrer Salze wird in der Weise durchgeführt, dass man den pH-Wert der wässrigen Lösung durch Zugabe einer Säure auf 1,5-4,5, vorzugsweise auf 3-4 stellt. Dabei kommen als Säuren

Mineralsäuren, wie Salzsäure, Schwefelsäure und Phosphorsäure, sowie organische Säuren, wie Ameisensäure und Essigsäure oder halogenierte Essigsäuren, wie Chloressigsäure, in Betracht. Vorzugsweise wird die Abscheidung der Salze der 2-(Imidazolin-2-yl)-pyridin- bzw. -chinolin-3-carbonsäuren der Formel I mit Salzsäure oder Schwefelsäure durchgeführt.

Die durch Zugabe von Säure aus den wässrigen Lösungen ihrer Salze abgeschiedenen freien 2-(Imidazolin-2-yl)-pyridin- bzw. -chinolin-3-carbonsäuren der Formel I können in der Regel durch Filtration oder Zentrifugation abgetrennt werden. Es ist jedoch auch möglich, die freien 2-(Imidazolin-2-yl)-pyridin- bzw. -chinolin-3-carbonsäuren durch Extraktion mit einem mit Wasser nicht mischbaren Lösungsmittel, beispielsweise einem halogenierten Kohlenwasserstoff, wie Methylenchlorid, Chloroform oder Kohlenstofftetrachlorid, und nachfolgendes Verdampfen des Lösungsmittels zu gewinnen. Diese Methode empfiehlt sich insbesondere dann, wenn die freien 2-(Imidazolin-2-yl)-pyridin- bzw. -chinolin-3-carbonsäuren der Formel I durch Zugabe von Säure nicht oder nicht vollständig aus der wässrigen Lösung ihrer Salze abgeschieden werden können. Die Methode empfiehlt sich ferner, wenn die wässrige Lösung der Salze der 2-(Imidazolin-2-yl)-pyridin- bzw. -chinolin-3-carbonsäuren der Formel I mit Wasser mischbare Lösungsmittel, wie niedere Alkanole, Tetrahydrofuran, Dioxan, Dimethylformamid und Dimethylsulfoxid enthält, die die Löslichkeit der freien Säuren in wässrigem Medium erhöhen können.

Bevorzugte 2-(Imidazolin-2-yl)-pyridin- und -chinolin-3-carbonsäuren der Formel I, welche nach dem erfindungsgemässen Verfahren hergestellt werden können, sind solche, in welchen $R_1$ Wasserstoff, $R_2$ und $R_3$ unabhängig voneinander je Wasserstoff, $C_1$-$C_2$-Alkyl, oder $R_2$ und $R_3$ zusammen 1,3-Butadienylen und $R_4$ und $R_5$ unabhängig voneinander je $C_1$-$C_3$-Alkyl bedeuten.

Besonders bevorzugte 2-(Imidazolin-2-yl)-pyridin- und -chinolin-3-carbonsäuren der Formel I sind solche, in welchen $R_1$ Wasserstoff, $R_2$ und $R_3$ unabhängig voneinander je $C_1$-$C_2$-Alkyl oder zusammen 1,3-Butadienylen, $R_4$ Methyl und $R_5$ Isopropyl bedeuten.

Die als bzw. in Resten $R_1$-$R_5$ vorkommenden $C_1$-$C_4$-Alkylreste können geradkettig oder verzweigt sein und umfassen Methyl, Ethyl, n-Propyl, Isopropyl, n-Butyl, sec.Butyl, Isobutyl und tert.Butyl. Dies gilt ebenfalls für Alkylreste die durch Halogen oder Phenyl substituiert sind. Die als bzw. in den Substituenten $R_1$-$R_6$ vorkommenden Phenylgruppen können einfach durch Halogen, $C_1$-$C_4$-Alkyl oder $C_1$-$C_4$-Halogenalkyl substituiert sein.

Eine bevorzugte Ausführungsform des erfindungsgemässen Verfahrens besteht darin, dass man die Umsetzung eines Pyridin- bzw. Chinolin-2,3-dicarbonsäureesters der Formel II mit einem 2-Aminoalkancarbonsäureamid der Formel III in Gegenwart von 1,5-2,5 Mol eines Alkalialkoholats pro Mol Diester der Formel II bei einer Temperatur von 50-90 °C in einem flüssigen aromatischen Kohlenwasserstoff oder Halogenkohlenwasserstoff durchführt, das abgeschiedene Salz der gebildeten 2-(Imidazolin-2-yl)-pyridin- bzw. -chinolin-3-carbonsäure der Formel I durch Zugabe von Wasser in eine wässrige Lösung überführt, die organische Phase abtrennt, die wässrige Phase durch Zugabe von wässriger Salzsäure oder wässriger Schwefelsäure auf einen pH-Wert von 3-4 stellt und die freie 2-(Imidazolin-2-yl)-pyridin- bzw. -chinolin-3-carbonsäure der Formel I abtrennt.

Die als Ausgangsmaterialien benötigten Pyridin-2,3-dicarbonsäureester der Formel II können in einfacher Weise durch Veresterung von Pyridin-2,3-dicarbonsäure mit entsprechenden Alkoholen hergestellt werden. Die Pyridin-2,3-dicarbonsäure kann ihrerseits durch Oxidation von Chinolin (vgl. DE-PS 1 010 524 und US-PS 2 512 482) hergestellt werden. Ferner können die Pyridin-2,3-dicarbonsäureester der Formel II nach der in den publizierten Europäischen Patentanmeldungen 0 161 221 und 0 172 140 beschriebenen Methoden ausgehend von einem Hydrazon einer $\alpha,\beta$-ungesättigten Carbonylverbindung und einem Maleinsäurederivat nach dem Schema einer Diels-Alder-Reaktion hergestellt werden. Die Chinolin-2,3-dicarbonsäureester der Formel II können nach der in J. Org. Chem. 49, 4999-5000, 1984 beschriebenen Methode erhalten werden. Die 2-Aminoalkancarbonsäureamide der Formel II sind grösstenteils bekannte Verbindungen, die in bekannter Weise durch Umsetzung entsprechender Ketone mit Ammoniak und Blausäure hergestellt werden können.

Das erfindungsgemässe Verfahren ermöglicht die Herstellung der 2-(Imidazolin-2-yl)-pyridin- und -chinolin-3-carbonsäuren der Formel I ausgehend von gut zugänglichen und leicht handhabbaren Ausgangsmaterialien in einfacher Weise und in guter Ausbeute. Im Gegensatz zu den bisher bekannten Verfahren werden die Salze der 2-(Imidazolin-2-yl)-pyridin- und -chinolin-3-carbonsäuren der Formel I in einem Reaktionsschritt erhalten. Ein weiterer Vorteil des erfindungsgemässen Verfahrens besteht darin, dass die gewünschten 2-(Imidazolin-2-yl)-pyridin- und -chinolin-3-carbonsäuren der Formel I in reiner Form frei von den Isomeren 3-(Imidazolin-2-yl)-pyridinund -chinolin-2-carbonsäuren erhalten werden.

Das erfindungsgemässe Verfahren wird durch die nachfolgenden Beispiele näher erläutert.

Beispiel 1: Herstellung von 2-(5-Isopropyl-5-methyl-4-oxoimidazolin-2-yl)-pyridin-3-carbonsäure

141,5 g (0,636 Mol) Pyridin-2,3-dicarbonsäurediethylester und 82,8 g (0,636 Mol) 2-Amino-2,3-dimethylbutyramid werden in 300 ml Toluol gelöst. Dann werden unter Rühren 160 g (1,4 Mol) Kalium-tert.butylat portionsweise eingetragen, wobei die Temperatur des Reaktionsgemisches auf 50-60 °C steigt. Nach beendigter Zugabe des Kaliumtert.butylats wird das Reaktionsgemisch noch 3 Stunden bei 80 °C gerührt, dann auf Raumtemperatur abgekühlt und das gebildete Kaliumsalz der 2-(5-Isopropyl-5-methyl-4-oxoimidazolin-2-yl)-pyridin-3-carbonsäure durch Filtration abgetrennt, 3 mal mit je 100 ml Aether gewaschen und in Wasser gelöst. Die erhaltene wässrige Lösung des Kaliumsalzes wird mit Kochsalz gesättigt und durch Zugabe von 37%iger Salzsäure auf pH 4 gestellt, wobei sich die freie 2-(5-Isopropyl-5-methyl-4-oxoimidazolin-2-yl)-pyridin-3-carbonsäure abscheidet. Das Produkt wird abfiltriert und in Methylenchlorid gelöst. Das Filtrat wird mit Methylenchlorid extrahiert und der Extrakt mit der Lösung des Produkts in Methylenchlorid vereinigt. Die vereinigten Methylenchlorid-Lösungen werden über Magnesiumsulfat getrocknet und das Lösungsmittel abdestilliert. Als Rückstand werden 132,8 g (80 % der Theorie) 2-(5-Isopropyl-5-methyl-4-oxoimidazolin-2-yl)-pyridin-3-carbonsäure vom Smp. 167-169 °C erhalten.

Beispiel 2: Herstellung von 2-(5-Isopropyl-5-methyl-4-oxoimidazolin-2-yl)-5-methylpyridin-3-carbonsäure

In eine Lösung von 123 g (0,519 Mol) 5-Methylpyridin-2,3-dicarbonsäurediethylester und 67,5 g (0,519 Mol) 2-Amino-2,3-dimethylbutyramid in 600 ml Toluol werden unter Rühren 116 g (1.034 Mol) Kalium-tert.butylat portionsweise eingetragen. Während der Zugabe des Kalium-tert.butylats erhöht sich die Temperatur der Mischung auf 60 °C. Nach beendigter Zugabe des Kalium-tert.butylats wird das Reaktionsgemisch auf 80 °C erwärmt und über Nacht gerührt. Dann werden der auf Raumtemperatur abgekühlten Mischung 800 ml Wasser zugesetzt und die Schichten getrennt. Die wässrige Phase wird durch Zugabe von 85 ml 37%iger Salzsäure auf pH 3 gestellt, auf 0 °C abgekühlt, filtriert und das erhaltene Produkt getrocknet. Es werden 97,8 g (69 % der Theorie) 2-(5-Isopropyl-5-methyl-4-oxoimidazolin-2-yl)-5-methylpyridin-3-carbonsäure vom Smp. 200-202 °C erhalten.

Beispiel 3: Herstellung von 2-(5-Isopropyl-5-methyl-4-oxoimidazolin-2-yl)-6-methylpyridin-3-carbonsäure

In eine Lösung von 41,8 g (0,2 Mol) 6-Methylpyridin-2,3-dicarbonsäuredimethylester und 26,6 g (0,2 Mol) 2-Amino-2,3-dimethylbutyramid in 600 ml Toluol werden bei Raumtemperatur unter Rühren 45 g (0,4 Mol) Kalium-tert.butylat eingetragen, wobei eine leicht exotherme Reaktion eintritt. Nach beendigter Zugabe des Kalium-tert.butylats wird das Reaktionsgemisch auf 90 °C erwärmt und 4 Stunden bei dieser Temperatur gerührt. Dann wird auf Raumtemperatur abgekühlt und das gebildete Kaliumsalz der 2-(5-Isopropyl-5-methyl-4-oxoimidazolin-2-yl)-6-methylpyridin-3-carbonsäure abfiltriert und getrocknet. Anschliessend wird das Kaliumsalz in Wasser gelöst und der pH-Wert der Lösung durch Zugabe von 37%iger Salzsäure auf 3 gestellt. Die erhaltene klare Lösung wird einmal mit Diethyläther und zweimal mit Methylenchlorid extrahiert. Die vereinigten Extrakte werden über Magnesiumsulfat getrocknet und eingedampft. Als Rückstand werden 30 g (54,5 % der Theorie) 2-(5-Isopropyl-5-methyl-4-oxoimidazolin-2-yl)-6-methylpyridin-3-carbonsäure vom Smp. 139-142 °C erhalten.

Beispiel 4: Herstellung von 2-(5-Isopropyl-5-methyl-4-oxoimidazolin-2-yl)-5-ethylpyridin-3-carbonsäure

In eine Lösung von 251 g (1 Mol) 5-Ethylpyridin-2,3-dicarbonsäurediethylesterund 130 g (1 Mol) 2-Amino-2,3-dimethylbutyramid in 1000 ml trockenem Toluol werden unter Rühren 225 g (2 Mol) Kalium-tert.butylat portionsweise eingetragen, wobei die Temperatur des Reaktiosgemisches auf 80 °C steigt. Die erhaltene rote Lösung wird nach beendigter Zugabe des Kalium-tert.butylats 4 Stunden bei 80 °C gerührt, wobei sich das Kaliumsalz der 2-(5-Isopropyl-5-methyl-4-oxoimidazolin-2-yl)-5-ethylpyridin-3-carbonsäure in Form eines dicken Kristallbreis abscheidet. Die auf Raumtemperatur abgekühlte Mischung wird nach Zugabe von 1000 ml Wasser bis zur vollständigen Auflösung des Kaliumsalzes gerührt. Anschliessend wird die organische Phase abgetrennt und die wässrige Phase durch Zugabe von 165 ml 37%iger Salzsäure auf pH 3 gestellt. Anschliessend wird die Mischung eine Stunde bei 0 °C gerührt und dann filtriert. Man erhält 214,4 g (74,2 % der Theorie) 2-(5-Isopropyl-5-methyl-4-oxoimidazolin-2-yl)-5-ethylpyridin-3-carbonsäure vom Smp. 171-173 °C.

Beispiel 5: Herstellung von 2-(5-Isopropyl-5-methyl-4-oxoimidazolin-2-yl)-5-brompyridin-3-carbonsäure

In eine Lösung von 22,0 g (0,073 Mol) 5-Brompyridin-2,3-dicarbonsäurediethylester und 9,5 g (0,073 Mol) 2-Amino-2,3-dimethylbutyramid in 250 ml Toluol werden unter Rühren 20,0 g (0,154 Mol) Kalium-tert.butylat eingetragen. Dann wird die erhaltene Mischung 6 Stunden bei 80 ° C gerührt und danach das gebildete Kaliumsalz der 2-(5-Isopropyl-5-methyl-4-oxoimidazolin-2-yl)-5-brompyridin-3-carbonsäure durch Filtration abgetrennt. Das Kaliumsalz wird in 150 ml Wasser gelöst und der pH-Wert der Lösung durch Zugabe von konzentrierter Salzsäure auf 3 gestellt. Danach wird die Mischung eine Stunde bei 0 ° C gerührt und filtriert. Es werden 15,5 (62,4 % der Theorie) 2-(5-Isopropyl-5-methyl-4-oxoimidazolin-2-yl)-5-brompyri din-3-carbonsäure vom Smp. 206-208 ° C erhalten.

Beispiel 6: Herstellung von 2-(5-Isopropyl-5-methyl-4-oxoimidazolin-2-yl)-chinolin-3-carbonsäure

90,0 g (0,33 Mol) Chinolin-2,3-dicarbonsäurediethylester und 43,0 g (0,33 Mol) 2-Amino-2,3-dimethylisobutyramid werden in 1 Liter Toluol gelöst. Dann werden 74,0 g (0,66 Mol) Kalium-tert. butylat unter starkem Rühren eingetragen, wobei die Temperatur auf 55 ° C steigt. Nach Zugabe des Kalium-tert.butylats wird das Reaktionsgemisch 5 Stunden bei 90 ° C gerührt, anschliessend auf Raumtemperatur abgekühlt und das gebildete Kaliumsalz der 2-(5-Isopropyl-5-methyl-4-oxoimidazolin-2-yl)-chinolin-3-carbonsäure durch Filtration abgetrennt. Das Produkt wird in 500 ml Wasser gelöst und der pH-Wert der Lösung durch Zugabe von konzentrierter Salzsäure auf 3 gestellt. Die gebildete Kristallsuspension wird eine Stunde bei 0 ° C gerührt und dann filtriert. Es werden 76 g (74 % der Theorie) 2-(5-Isopropyl-5-methyl-4-oxoimidazolin-2-yl)-chinolin-3-carbonsäure vom Smp. 266-270 ° C erhalten.

Auf analoge Weise werden ausgehend von 21,5 g (0,07 Mol) 6-Chlorchinolin-2,3-dicarbonsäurediethylester und 9,1 g (0,07 Mol) 2-Amino-2,3-dimethylbutyramid und 17,0 g (0,15 Mol) Kalium-tert. butylat 19,9 g (82,3 % der Theorie) 2-(5-Isopropyl-5-methyl-4-oxoimidazolin-2-yl)-6-chlorchinolin-3-carbonsäure vom Smp. 234-237 ° C erhalten.

## Patentansprüche

**1.** Verfahren zur Herstellung von 2-(Imidazolin-2-yl)-pyridin- und -chinolin-3-carbonsäuren der Formel I

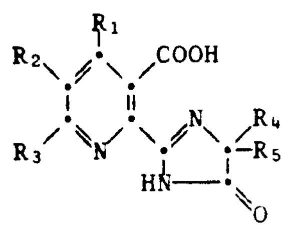

(I)

in welcher $R_1$ Wasserstoff oder $C_1$-$C_4$-Alkyl, $R_2$ Wasserstoff, Halogen, $C_1$-$C_4$-Alkyl, Hydroxy-$C_1$-$C_4$-alkyl, $C_1$-$C_4$-Alkoxy, Phenyl, oder Phenyl-$C_1$-$C_4$-alkyl oder einfach durch $C_1$-$C_4$-Alkyl-$C_1$-$C_4$-alkoxy oder Halogen substituiertes Phenyl oder Phenyl-$C_1$-$C_4$-alkyl, $R_3$ Wasserstoff, $C_1$-$C_4$-Alkyl, Phenyl, Phenyl-$C_1$-$C_4$-alkyl oder durch $C_1$-$C_4$-Alkyl, $C_1$-$C_4$-Alkoxy oder Halogen einfach substituiertes Phenyl oder Phenyl-$C_1$-$C_4$-alkyl, $R_2$ und $R_3$ zusammen 1,3-Butadienylen, das durch Halogen, $C_1$-$C_4$-Alkyl, $C_1$-$C_4$-Alkoxy, $C_1$-$C_4$-Halogenalkyl, $C_1$-$C_4$-Alkylsulfonyl, Nitro, Cyano, Phenyl, Phenoxy oder durch $C_1$-$C_4$-Alkyl, $C_1$-$C_4$-Alkoxy oder Halogen einfach substituiertes Phenyl oder Phenoxy substituiert sein kann und $R_4$ und $R_5$ unabhängig voneinander $C_1$-$C_4$-Alkyl bedeuten, dadurch gekennzeichnet, dass man einen Pyridin- oder Chinolin-2,3-dicarbonsäureester der Formel II

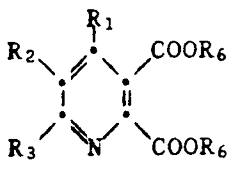

(II)

in welcher $R_1$, $R_2$ und $R_3$ die unter Formel I angegebene Bedeutung haben und $R_6$ $C_1$-$C_8$-Alkyl, Phenyl oder Phenyl-$C_1$-$C_4$-alkyl bedeuten, in einem inerten Lösungsmittel in Gegenwart einer starken Base bei einer Temperatur von Raumtemperatur bis Rückflusstemperatur des Reaktionsgemisches mit einem 2-Aminoalkancarbonsäureamid der Formel III

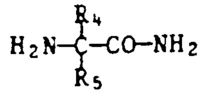 (III)

in welcher $R_4$ und $R_5$ die unter Formel I angegebene Bedeutung haben, umsetzt, das gebildete Salz der 2-(Imidazolin-2-yl)-pyridin- oder -chinolin-3-carbonsäure der Formel I mit Wasser aufnimmt, den pH-Wert der wässrigen Lösung auf 1,5-4,5 stellt und die freie 2-(Imidazolin-2-yl)-pyridin- oder -chinolin-3-carbonsäure der Formel I abtrennt.

**2.** Verfahren nach Anspruch 1, dadurch gekennzeichnet, dass man als inertes Lösungsmittel einen flüssigen aromatischen Kohlenwasserstoff oder Halogenkohlenwasserstoff, ein $C_1$-$C_{10}$-Alkanol, Tetrahydrofuran, Dioxan, Acetonitril, N,N-Dimethylformamid oder Dimethylsulfoxid verwendet.

**3.** Verfahren nach Anspruch 1, dadurch gekennzeichnet, dass man als inertes Lösungsmittel Benzol, Toluol, Chlorbenzol oder ein Xylol verwendet.

**4.** Verfahren nach Anspruch 1, dadurch gekennzeichnet, dass man die Umsetzung eines Pyridin- oder Chinolin-2,3-dicarbonsäureesters der Formel II mit einem 2-Aminoalkancarbonsäureamid der Formel III in Gegenwart eines Alkalimetallhydroxids oder Alkalimetallalkoholats als starke Base durchführt.

**5.** Verfahren nach Anspruch 1, dadurch gekennzeichnet, dass man die Umsetzung eines Pyridin- oder Chinolin-2,3-dicarbonsäureesters der Formel II mit einem 2-Aminoalkancarbonsäureamid der Formel III in Gegenwart eines von einem $C_1$-$C_4$-Alkanol abgeleiteten Alkalimetallalkoholats durchführt.

**6.** Verfahren nach Anspruch 5, dadurch gekennzeichnet, dass man die Umsetzung eines Pyridin- oder Chinolin-2,3-dicarbonsäureesters der Formel II mit einem 2-Aminoalkancarbonsäureamid der Formel III in Gegenwart von Kalium-tert.butylat durchführt.

**7.** Verfahren nach Ansprüchen 1 und 4, dadurch gekennzeichnet, dass man 1,5-2,5 Mol Base pro Mol Pyridin- bzw. Chinolin-2,3-dicarbonsäureester der Formel II verwendet.

**8.** Verfahren nach Anspruch 1, dadurch gekennzeichnet, dass man die Umsetzung eines Pyridin- oder Chinolin-2,3-dicarbonsäureesters der Formel II mit einem 2-Aminoalkancarbonsäureamid der Formel III bei einer Temperatur von 50-90 ° C durchführt.

**9.** Verfahren nach Anspruch 1, dadurch gekennzeichnet, dass man das bei der Umsetzung eines Pyridin- oder Chinolin-2,3-dicarbonsäureesters der Formel II mit einem 2-Aminoalkancarbonsäureamid der Formel III gebildete Salz einer 2-(Imidazolin-2-yl)-pyridin- oder -chinolin-3-carbonsäure der Formel I durch Filtration aus dem Reaktionsgemisch abtrennt und durch Auflösen in Wasser in eine wässrige Lösung überführt.

**10.** Verfahren nach Anspruch 1, dadurch gekennzeichnet, dass man das durch Umsetzung eines Pyridin- oder -Chinolin-2,3-dicarbonsäureesters der Formel II mit einem 2-Aminoalkancarbonsäureamid der Formel III gebildete Salz einer 2-(-Imidazolin-2-yl)-pyridin- oder -chinolin-3-carbonsäure der Formel I durch direkte Zugabe von Wasser zum Reaktionsgemisch in eine wässrige Lösung überführt.

**11.** Verfahren nach Anspruch 1, dadurch gekennzeichnet, dass man die 2-(Imidazolin-2-yl)-pyridin- oder -chinolin-3-carbonsäuren der Formel I aus der wässrigen Lösung ihrer Salze freisetzt, indem man den pH-Wert der wässrigen Lösung durch Zugabe von Salzsäure oder Schwefelsäure auf 3-4 stellt.

**12.** Verfahren nach Anspruch 1, dadurch gekennzeichnet, dass man 2-(Imidazolin-2-yl)-pyridin- oder -chinolin-3-carbonsäuren der Formel I herstellt, in welchen $R_1$ Wasserstoff, $R_2$ und $R_3$ unabhängig voneinander je Wasserstoff, $C_1$-$C_2$-Alkyl, oder $R_2$ und $R_3$ zusammen 1,3-Butadienylen und $R_4$ und $R_5$ unabhängig voneinander je $C_1$-$C_3$-Alkyl bedeuten.

**13.** Verfahren nach Anspruch 1, dadurch gekennzeichnet, dass man 2-(Imidazolin-2-yl)-pyridin- oder -chinolin-3-carbonsäuren der Formel I herstellt, in welchen $R_1$ Wasserstoff, $R_2$ und $R_3$ unabhängig

voneinander je $C_1$-$C_2$-Alkyl oder zusammen 1,3-Butadien-ylen, $R_4$ Methyl und $R_5$ Isopropyl bedeuten.

**14.** Verfahren nach Anspruch 1, dadurch gekennzeichnet, dass man die Umsetzung eines Pyridin- oder -Chinolin-2,3-dicarbonsäureester der Formel II mit einem 2-Aminoalkancarbonsäureamid der Formel III in Gegenwart von 1,5-2,5 Mol eines Alkalialkoholats pro Mol Diester der Formel II bei einer Temperatur von 50-90 °C in einem flüssigen aromatischen Kohlenwasserstoff oder Halogenkohlenwasserstoff durchführt, das abgeschiedene Salz der gebildeten 2-(Imidazolin-2-yl)-pyridin- bzw. -chinolin-3-carbonsäure der Formel I durch Zugabe von Wasser in eine wässrige Lösung überführt, die organische Phase abtrennt, die wässrige Phase durch Zugabe von wässriger Salzsäure oder wässriger Schwefelsäure auf einen pH-Wert von 3-4 stellt und die freie 2-(Imidazolin-2-yl)-pyridin- bzw. -chinolin-3-carbonsäure der Formel I abtrennt.

## Claims

**1.** A process for the preparation of a 2-(imidazolin-2-yl)pyridine- or-quinoline-3-carboxylic acid of formula I

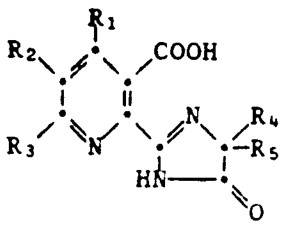

(I)

wherein $R_1$ is hydrogen or $C_1$-$C_4$alkyl, $R_2$ is hydrogen, halogen, $C_1$-$C_4$alkyl, hydroxy-$C_1$-$C_4$alkyl, $C_1$-$C_4$alkoxy, phenyl or phenyl-$C_1$-$C_4$alkyl, or phenyl or phenyl-$C_1$-$C_4$alkyl each substituted by one $C_1$-$C_4$alkyl-$C_1$-$C_4$alkoxy or halogen, $R_3$ is hydrogen, $C_1$-$C_4$alkyl, phenyl, phenyl-$C_1$-$C_4$alkyl, or phenyl or phenyl-$C_1$-$C_4$alkyl each substituted by one $C_1$-$C_4$alkyl, $C_1$-$C_4$alkoxy or halogen, $R_2$ and $R_3$ together are 1,3-butadienylene which can be substituted by halogen, $C_1$-$C_4$alkyl, $C_1$-$C_4$alkoxy, $C_1$-$C_4$haloalkyl, $C_1$-$C_4$alkylsulfonyl, nitro, cyano, phenyl, phenoxy, or phenyl or phenoxy each substituted by one $C_1$-$C_4$alkyl, $C_1$-$C_4$alkoxy or halogen, and $R_4$ and $R_5$ are each independently of the other $C_1$-$C_4$alkyl, which process comprises reacting a pyridine- or -quinoline-2,3-dicarboxylic acid ester of formula II

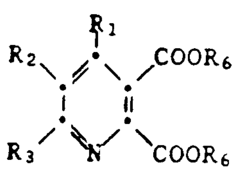

(II)

wherein $R_1$, $R_2$ and $R_3$ are as defined for formula I, and $R_6$ is $C_1$-$C_8$alkyl, phenyl or phenyl-$C_1$-$C_4$alkyl, with a 2-aminoalkanecarboxamide of formula III

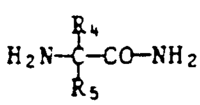

(III)

wherein $R_4$ and $R_5$ are as defined for formula I, in an inert solvent and in the presence of a strong base, at a temperature in the range from room temperature to the reflux temperature of the reaction mixture, dissolving the resultant salt of the 2-(imidazolin-2-yl)pyridine- or -quinoline-3-carboxylic acid of formula I in water, adjusting the pH of the aqueous solution to 1.5 - 4.5, and isolating the free 2-(imidazolin-2-yl)pyridine- or -quinoline-3-carboxylic acid of formula I.

**2.** A process according to claim 1, wherein the inert solvent used is a liquid aromatic hydrocarbon or halohydrocarbon, a $C_1$-$C_{10}$alkanol, tetrahydrofuran, dioxane, acetonitrile, N,N-dimethylformamide or dimethyl sulfoxide.

**3.** A process according to claim 1, wherein the inert solvent used is benzene, toluene, chlorobenzene or a xylene.

**4.** A process according to claim 1, wherein the reaction of a pyridine-or quinoline-2,3-dicarboxylic acid ester of formula II with a 2-aminoalkanecarboxamide of formula III is carried out in the presence of an alkali metal hydroxide or alkali metal alcoholate as strong base.

**5.** A process according to claim 1, wherein the reaction of a pyridine-or quinoline-2,3-dicarboxylic acid ester of formula II with a 2-aminoalkanecarboxamide of formula III is carried out in the presence of an alkali metal alcoholate which is derived from a $C_1$-$C_4$alkanol.

**6.** A process according to claim 5, wherein the reaction of a pyridine-or quinoline-2,3-dicarboxylic acid ester of formula II with a 2-aminoalkanecarboxamide of formula III is carried out in the presence of potassium tert-butylate.

**7.** A process according to either of claims 1 or 4, wherein 1.5 to 2.5 mol of base are used per mole of pyridine- or quinoline-2,3-dicarboxylic acid ester of formula II.

**8.** A process according to claim 1, wherein the reaction of a pyridine-or quinoline-2,3-dicarboxylic acid ester of formula II with a 2-aminoalkanecarboxamide of formula III is carried out at a temperature from 50 to 90 ° C.

**9.** A process according to claim 1, wherein the salt of a 2-(imidazolin-2-yl)pyridine- or -quinoline-3-carboxylic acid of formula I, obtained by reacting a pyridine- or quinoline-2,3-dicarboxylic acid ester of formula II with a 2-aminoalkanecarboxamide of formula III, is isolated by filtration from the reaction mixture and converted into an aqueous solution by dissolution in water.

**10.** A process according to claim 1, wherein the salt of a 2-(imidazolin-2-yl)pyridine- or -quinoline-3-carboxylic acid of formula I, obtained by reacting a pyridine- or -quinoline-2,3-dicarboxylic acid ester of formula II with a 2-aminoalkanecarboxamide of formula III, is converted into an aqueous solution by direct addition of water to the reaction mixture.

**11.** A process according to claim 1, wherein the 2-(imidazolin-2-yl)pyridine- or -quinoline-3-carboxylic acid of formula I is recovered from the aqueous solution of its salt by adjusting the pH of the aqueous solution to 3 - 4 by addition of hydrochloric acid or sulfuric acid.

**12.** A process according to claim 1, which comprises preparing a 2-(imidazolin-2-yl)pyridine- or -quinoline-3-carboxylic acid of formula I wherein $R_1$ is hydrogen, $R_2$ and $R_3$ are each independently of the other hydrogen or $C_1$-$C_2$alkyl or $R_2$ and $R_3$ together are 1,3-butadienylene, and $R_4$ and $R_5$ are each independently of the other $C_1$-$C_3$alkyl.

**13.** A process according to claim 1, which comprises preparing a 2-(imidazolin-2-yl)pyridine- or -quinoline-3-carboxylic acid of formula I wherein $R_1$ is hydrogen, $R_2$ and $R_3$ are each independently of the other $C_1$-$C_2$alkyl or together are 1,3-butadienylene, $R_4$ is methyl and $R_5$ is isopropyl.

**14.** A process according to claim 1, which comprises carrying out the reaction of a pyridine- or -quinoline-2,3-dicarboxylic acid ester of formula II with a 2-aminoalkanecarboxamide of formula III in the presence of 1.5 to 2.5 mol of an alkali metal alcoholate per mole of diester of formula II, at a temperature from 50 to 90 ° C, and in a liquid aromatic hydrocarbon or halohydrocarbon, converting the separated salt of the resultant 2-(imidazolin-2-yl)pyridine- or -quinoline-3-carboxylic acid of formula I into an aqueous solution by addition of water, isolating the organic phase, adjusting the pH of the aqueous phase to 3 - 4 by addition of aqueous hydrochloric acid or aqueous sulfuric acid, and isolating the free 2-(imidazolin-2-yl)-pyridine- or -quinoline-3-carboxylic acid of formula I.

**Revendications**

**1.** Procédé de préparation des acides 2-(imidazoline-2-yl)-pyridine et -quinoléine-3-carboxyliques de formule I

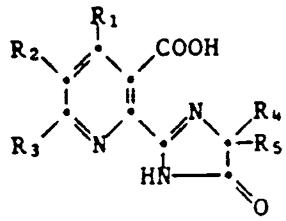

(I)

dans laquelle $R_1$ représente l'hydrogène ou un groupe alkyle en C1-C4, $R_2$ représente l'hydrogène, un halogène, un groupe alkyle en C1-C4, hydroxyalkyle en C1-C4, alcoxy en C1-C4, phényle ou phényl-alkyle en C1-C4, ou un groupe phényle ou phényl-alkyle en C1-C4 portant un substituant (alkyle en C1-C4)-alcoxy en C1-C4 ou halogéno, $R_3$ représente l'hydrogène, un groupe alkyle en C1-C4, phényle, phényl-alkyle en C1-C4 ou un groupe phényle ou phényl-alkyle en C1-C4 portant un substituant alkyle en C1-C4, alcoxy en C1-C4 ou halogéno, $R_2$ et $R_3$ forment ensemble un groupe 1,3-butadiénylène qui peut être substitué par des halogènes, des groupes alkyle en C1-C4, alcoxy en C1-C4, halogénoalkyle en C1-C4, alkylsulfonyle en C1-C4, nitro, cyano, phényle, phénoxy, ou par des groupes phényle ou phénoxy portant eux-mêmes un substituant alkyle en C1-C4, alcoxy en C1-C4 ou halogéno, et $R_4$ et $R_5$ représentent chacun, indépendamment l'un de l'autre, un groupe alkyle en C1-C4, caractérisé en ce que l'on fait réagir un ester d'acide pyridine- ou quinoléine-2,3-dicarboxylique de formule II

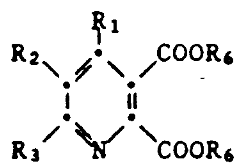

(II)

dans laquelle $R_1$, $R_2$ et $R_3$ ont les significations indiquées en référence à la formule I et $R_6$ représente un groupe alkyle en C1-C8, phényle ou phényl-alkyle en C1-C4, dans un solvant inerte, en présence d'une base forte, à une température comprise entre la température ambiante et la température de reflux du mélange de réaction, avec un amide d'acide 2-aminoalcanoïque de formule III

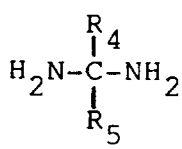

(III)

dans laquelle $R_4$ et $R_5$ ont les significations indiquées en référence à la formule I, ce qui donne un sel de l'acide 2-(imidazoline-2-yl)-pyridine- ou -quinoléine-3-carboxylique de formule I qu'on reprend à l'eau, on règle la solution aqueuse à un pH de 1,5 à 4,5 et on sépare l'acide 2-(imidazoline-2-yl)-pyridine- ou -quinoléine-3-carboxylique libre de formule I.

**2.** Procédé selon la revendication 1, caractérisé en ce que l'on utilise en tant que solvant inerte un hydrocarbure aromatique ou un hydrocarbure halogéné liquide, un alcanol en C1-C10, le tétrahydrofuranne, le dioxanne, l'acétonitrile, le N,N-diméthylformamide ou le diméthylsulfoxyde.

**3.** Procédé selon la revendication 1, caractérisé en ce que l'on utilise en tant que solvant inerte le benzène, le toluène, le chlorobenzène ou un xylène.

**4.** Procédé selon la revendication 1, caractérisé en ce que la réaction entre un ester d'acide pyridine-ou quinoléine-2,3-dicarboxylique de formule II et un amide d'acide 2-amino-alcanoïque de formule III est effectuée en présence d'une base forte consistant en un hydroxyde de métal alcalin ou un alcoolate de métal alcalin.

**5.** Procédé selon la revendication 1, caractérisé en ce que la réaction entre un ester d'acide pyridine-ou quinoléine-2,3-dicarboxylique de formule II et un amide d'acide 2-aminoalcanoïque de formule III est

effectuée en présence d'un alcoolate de métal alcalin dérivant d'un alcanol en C1-C4.

6. Procédé selon la revendication 5, caractérisé en ce que la réaction entre un ester d'acide pyridine-ou quinoléine-2,3-dicarboxylique de formule II et un amide d'acide 2-aminoalcanoïque de formule III est effectuée en présence du tert-butylate de potassium.

7. Procédé selon les revendications 1 et 4, caractérisé en ce que l'on utilise de 1,5 à 2,5 mol de la base par mol de l'ester pyridine- ou quinoléine-2,3-dicarboxylique de formule II.

8. Procédé selon la revendication 1, caractérisé en ce que la réaction entre un ester d'acide pyridine-ou quinoléine-2,3-dicarboxylique de formule II et un amide d'acide 2-amino-alcanoïque de formule III est effectuée à une température de 50 à 90 ° C.

9. Procédé selon la revendication 1, caractérisé en ce que le sel d'un acide 2-(imidazoline-2-yl)-pyridine- ou -quinoléine-3-carboxylique de formule I formé à la réaction entre un ester d'acide pyridine- ou quinoléine-2,3-dicarboxylique de formule II et un amide d'acide 2-aminoalcanoïque de formule III est séparé du mélange de réaction par filtration et mis à l'état de solution aqueuse par redissolution dans l'eau.

10. Procédé selon la revendication 1, caractérisé en ce que le sel d'acide 2-(imidazoline-2-yl)-pyridine-ou -quinoléine-3-carboxylique de formule I formé à la réaction entre un ester d'acide pyridine- ou quinoléine-2,3-dicarboxylique de formule II et un amide d'acide 2-aminoalcanoïque de formule III est mis à l'état de solution aqueuse par addition directe d'eau au mélange de réaction.

11. Procédé selon la revendication 1, caractérisé en ce que les acides 2-(imidazoline-2-yl)-pyridine- ou -quinoléine-3-carboxyliques de formule I sont libérés de la solution aqueuse de leurs sels par réglage du pH de cette solution aqueuse à un niveau de 3 à 4 par addition d'acide chlorhydrique ou sulfurique.

12. Procédé selon la revendication 1, caractérisé en ce que l'on prépare les acides 2-(imidazoline-2-yl)-pyridine- ou -quinoléine-3-carboxyliques de formule I pour lesquels $R_1$ représente l'hydrogène, $R_2$ et $R_3$ représentent chacun, indépendamment l'un de l'autre, l'hydrogène, un groupe alkyle en C1-C2, ou bien $R_2$ et $R_3$ forment ensemble un groupe 1,3-butadiénylène, et $R_4$ et $R_5$ représentent chacun, indépendamment l'un de l'autre, un groupe alkyle en C1-C3.

13. Procédé selon la revendication 1, caractérisé en ce que l'on prépare les acides 2-(imidazoline-2-yl)-pyridine- ou -quinoléine-3-carboxyliques de formule I pour lesquels $R_1$ représente l'hydrogène, $R_2$ et $R_3$ représentent chacun, indépendamment l'un de l'autre, un groupe alkyle en C1-C2 ou bien forment ensemble un groupe 1,3-butadiénylène, $R_4$ représente un groupe méthyle et $R_5$ un groupe isopropyle.

14. Procédé selon la revendication 1, caractérisé en ce que la réaction entre un ester d'acide pyridine- ou quinoléine-2,3-dicarboxylique de formule II et un amide d'acide 2-amino-alcanoïque de formule III est effectuée en présence de 1,5 à 2,5 mol d'un alcoolate alcalin par mol du diester de formule II a une température de 50 à 90 ° C dans un hydrocarbure aromatique ou un hydrocarbure halogéné liquide, le sel de l'acide 2-(imidazoline-2-yl)-pyridine- ou -quinoléine-3-carboxylique de formule I formé et qui s'est séparé est mis à l'état de solution aqueuse par addition d'eau, la phase organique est séparée, la phase aqueuse est réglée à pH 3-4 par addition d'acide chlorhydrique aqueux ou d'acide sulfurique aqueux, et l'acide 2-(imidazoline-2-yl)-pyridine- ou -quinoléine-3-carboxylique libre de formule I est isolé.